# EUROPEAN PATENT SPECIFICATION

(11) **EP 2 794 861 B1**
(45) Date of publication and mention of the grant of the patent: **14.11.2018**
(21) Application number: 12821295.8
(22) Date of filing: 21.12.2012
(51) Int. Cl.: C12N 7/00, H01S 3/02, H01S 3/213

(54) **A COMPOSITION**
ZUSAMMENSETZUNG
COMPOSITION

(30) Priority: 22.12.2011 GB 201122158
(43) Date of publication of application: 29.10.2014
(73) Proprietor: UCL Business Plc., London W1T 4TP (GB)
(72) Inventor: HALES, John Edward, London WC1E 6BT (GB); WARD, John, London WC1E 6BT (GB); AEPPLI, Gabriel, London WC1E 6BT (GB); DAFFORN, Tim, Edgbaston Birmingham B15 2TT (GB)
(74) Representative: Hutter, Anton
(86) International application number: PCT/GB2012/053236
(87) International publication number: WO 2013/093499

(56) References cited:
- WO-A2-2005/034997
- WO-A2-2005/034997
- US-A- 6 141 367
- US-A1- 2010 190 233
- US-A1- 2010 190 233
- US-A1- 2011 266 470
- KAI LI ET AL: "Chemical Modification of M13 Bacteriophage and Its Application in Cancer Cell Imaging", BIOCONJUGATE CHEMISTRY, vol. 21, no. 7, 21 July 2010 (2010-07-21), pages 1369-1377, XP055056043, ISSN: 1043-1802, DOI: 10.1021/bc900405q
- JAYE D L ET AL: "Direct fluorochrome labeling of phage display library clones for studying binding specificities: applications in flow cytometry and fluorescence microscopy", JOURNAL OF IMMUNOLOGICAL METHODS, vol. 295, no. 1-2, 1 December 2004 (2004-12-01), pages 119-127, XP004701198, ELSEVIER SCIENCE PUBLISHERS B.V.,AMSTERDAM, NL ISSN: 0022-1759, DOI: 10.1016/J.JIM.2004.09.011
- KELLY KIMBERLY A ET AL: "In vivo imaging of molecularly targeted phage", NEOPLASIA (NEW YORK), vol. 8, no. 12, December 2006 (2006-12), pages 1011-1018, XP002693723, ISSN: 1522-8002
- PIURI MARIANA ET AL: "Fluoromycobacteriophages for rapid, specific, and sensitive antibiotic susceptibility testing of Mycobacterium tuberculosis.", PLOS ONE, vol. 4, no. 3, E4870, 2009, pages 1-12, XP002693724, ISSN: 1932-6203
- Kai Li ET AL: "Chemical Modification of M13 Bacteriophage and Its Application in Cancer Cell Imaging", Bioconjugate Chemistry, vol. 21, no. 7, 21 July 2010 (2010-07-21), pages 1369-1377, XP055056043, ISSN: 1043-1802, DOI: 10.1021/bc900405q
- JAYE D L ET AL: "Direct fluorochrome labeling of phage display library clones for studying binding specificities: applications in flow cytometry and fluorescence microscopy", JOURNAL OF IMMUNOLOGICAL METHODS, ELSEVIER SCIENCE PUBLISHERS B.V.,AMSTERDAM, NL, vol. 295, no. 1-2, 1 December 2004 (2004-12-01), pages 119-127, XP004701198, ISSN: 0022-1759, DOI: 10.1016/J.JIM.2004.09.011
- KIMBERLY A KELLY ET AL: "In vivo imaging of molecularly targeted phage", NEOPLASIA, NEOPLASIA PRESS, ANN ARBOR, MI, US, vol. 8, no. 12, 1 December 2006 (2006-12-01), pages 1011-1018, XP002693723, ISSN: 1522-8002, DOI: 10.1593/NEO.06610
- MARIANA PIURI ET AL: "Fluoromycobacteriophages for rapid, specific, and sensitive antibiotic susceptibility testing of Mycobacterium tuberculosis", PLOS ONE, PUBLIC LIBRARY OF SCIENCE, US, vol. 4, no. 3, 20 March 2009 (2009-03-20), pages e4870-1, XP002693724, ISSN: 1932-6203, DOI: 10.1371/JOURNAL.PONE.0004870 [retrieved on 2009-03-20]
- TANIGUCHI H ET AL: "Biological Laser Emission Achieved By Mixed Highly Scattering Intralipid-10% In Microdroplets Including Pigment Extracted From Green Algae", LASERS AND ELECTRO-OPTICS, 1997. CLEO/PACIFIC RIM '97., PACIFIC RIM CO NFERENCE ON CHIBA, JAPAN 14-18 JULY 1997, NEW YORK, NY, USA,IEEE, US, 14 July 1997 (1997-07-14), pages 156-157, XP032306014, DOI: 10.1109/CLEOPR.1997.610669 ISBN: 978-0-7803-3889-0
- DICE G ET AL: "Plasmonically enhanced diffusive and subdiffusive metal nanoparticle-dye random laser", APPLIED PHYSICS LETTERS, A I P PUBLISHING LLC, US, vol. 86, no. 13, 21 March 2005 (2005-03-21), pages 131105-131105, XP012064790, ISSN: 0003-6951, DOI: 10.1063/1.1894590

## Description

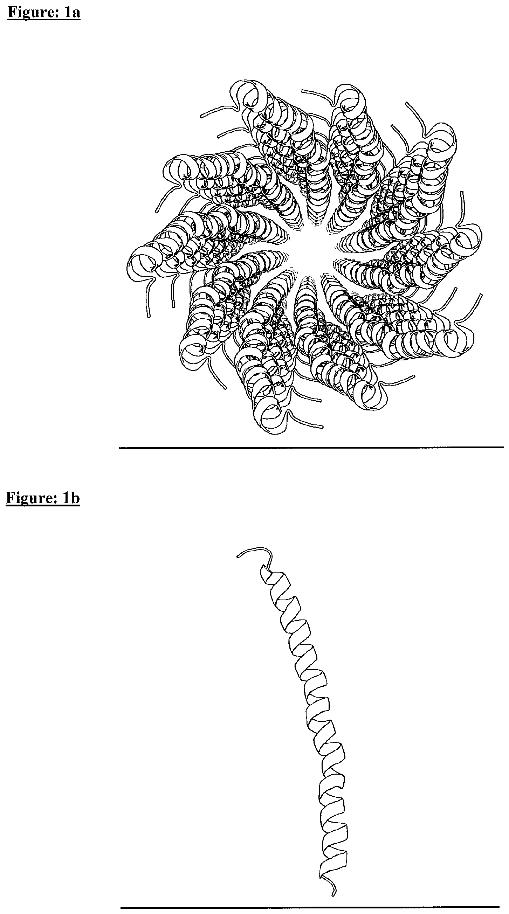

The present invention relates to lasing compositions. The invention particularly relates to programmable biological substrates, which lase, and which have a wide variety of different applications. The invention extends to use of the lasing compositions comprising programmable biological substrates in fabricating lasers, and in various biological imaging applications, such as in assays.
Structural biology has shown that the function of molecular machines, including proteins and RNAzymes, is determined by their structure and that structural details on the angstrom scale can be decisive. The structure and consequently the function of a protein can be modified by mutations to the gene coding for the protein or by post-translational modifications to the protein surface. As such, biology at the molecular scale is readily programmable because new or modified functionality can be conferred to biological systems by making genetic mutations or chemical modifications to proteins.
Scientists engaged in the field of nanotechnology look to make novel devices on the scale of nanometres, which is the same length scale as molecular biology. These scientists have begun looking towards the programmable nature of biology to take advantage of biological structure and complexity. So far, the interface between molecular biology and nanotechnology has been centred on the development of virus hybrid systems. Examples of virus hybrid devices include lithium batteries, cobalt wires, light harvesting devices, magnetic memory storage devices and ordered arrays of quantum dots in viral films.
M13 has been the substrate of choice for much of this research because it has a number of desirable attributes. M13 will only infect F' pilus strains of *E. coli* and typical yields from overnight shakeflask production are of the order 5 x 10¹¹ phage per ml. The wild type phage forms long, thin rod like particles with dimensions 9300 Å by 65 Å, as shown in Figure 1, but the length can be controlled by varying die length of the genome or by altering the number of positively charged residues at the C-terminal end of die major coat protein. In the wild type phage, there are 2700 copies of the major coat protein, g8p, packaging a ssDNA genome of 6407 bases. The major coat protein is 50 amino acids long and its secondary structure is an alpha helix with an unstructured N-terminus. The C-terminus is buried behind other coat proteins and the N-terminus is accessible to the bulk solvent. There are five copies of the minor coat proteins, g3p, g6p, g7p and g9p. Each of the M13 structural proteins can be mutated to add new functionality to the surface at either end or side, making it an excellent candidate for engineering. The literature surrounding the molecular biology of M13 is extensive so some modifications that will disrupt the assembly of the phage can be avoided.

Without prior genetic mutation, the major coat proteins of M13 can be modified by direct functionalization of the amino (N-terminus and lysine), carboxylic acid (glutamic acid and aspartic acid) and phenolic (tyrosine) groups exposed at the surface. The pKₐ of the amino group at the N-terminus is 7.6-8.0, but the pKₐ of the lysine amino group is 9.3-9..5. As such, the N-terminus can be selectively modified in reactions buffered at lower pH. M13 has been found to be stable when incubated in solutions of pH 3.0 to 11.0 and when heated to temperatures of 80°C. M13 can be exposed to concentrations of at least 20% methanol, ethanol, 1-propanol, acetonitrile or N, N-dimethylformamide and remain infective. Often M13 can be exposed to much higher concentrations of solvent. For instance, helper phage strain pG8H6 can be exposed to 99% acetonitrile.

The optical properties of rod-like viruses modified with dyes and catalysts have been studied with a view to making light harvesting devices. The practice of making nanoantennae from biological scaffolds represents an effort to use biology in a domain usually dominated by physicists. The transfer of energy between dyes on biological scaffolds via FRET is an established phenomenon. Accordingly, time-resolved fluorescence spectroscopy of these systems reveals a reduction in the lifetime of the excited state of the scaffolded dyes. The overall fluorescence intensity of M13 labelled with N,N,N',N'-tetramethylrhodamine or Zn(II)-porphyrin derivatives decreases as more dyes are conjugated to the surface because of the close proximity of dyes labelled to the N-terminus and lysine residues of the same coat protein. However, in other systems labelling does not lead to fluorescence quenching. For instance, another virus, cowpea mosaic virus (CPMV), was labelled with fluorescein derivatives to a high density with no observable quenching and used for intravital vascular imaging. Rhodamine and porphyrin molecules are both bulkier than fluorescein molecules so their wavefunctions are more likely to overlap on the surface of biological scaffolds when there is a high density of conjugated dyes. A more common practice is to use dye-labelled viruses in biosensors or biological assays. For these practices, the physics of the dyes labelled to the biological substrate is not the primary concern of the research.

Filamentous bacteriophage fd, a close relative of M13, has been labelled with fluorescein to measure the self diffusion of rod-like viruses in the nematic phase using fluorescence microscopy. The labelled viruses were observed to be relatively photostable, though this is attributed to the mixing of the samples in an anti-oxygen solution. In the biosensor field, fluorescently stained LG1 phage and a PP01 phage displaying green fluorescent protein fusions have been used to detect *E*. *coli* O157:H7 cells. Labelled fd can be taken up by human B cells and their localization observed with confocal microscopy. Additionally, MS2 bacteriophage has been labelled with dyes and used as a tracer and a T7 phage was hybridized with a europium complex to create a new fluorescent probe for imaging and bioassays. Fluorescently stained viruses can be used to identify specific bacterial strains in a microbial community.

The most common use of phage in biotechnology is phage display, which is a well-established method of finding peptide sequences with an affinity to a target. Fusion proteins can be expressed on the surface of M13 phage by direct genetic modification of the phage genome or by transforming cells with a phagemid and infecting them with helper phage. Phage display to discover peptides that bind non-biological targets, including GaAs, ZnS, CdS, PbS, Au, Ag, Co and carbon nanotubes, has proven successful and phage display to identify antibody fragments is routine.

Once phage display has identified a phage clone with an affinity to a target, it is possible to use the phage as though it was a primary antibody in immunobiological assays using a conjugated anti-M13 secondary antibody. Alternatively, the phage itself can be fluorescently labelled. For instance, T7 can be genetically engineered to display enhanced yellow fluorescent protein fusions and M13 has been labelled with a fluorescein derivative. The M13 derivatised with fluorescein may have had a structure similar to M13-fluorophage, but no evidence was presented that the construct had enhanced optical properties. The construct did retain its capacity to bind to targets and so could be used as a primary phage conjugate. Horse radish peroxidase (HRP) conjugated protein A is one alternative to secondary antibodies in immunobiological assays because all of the domains of protein A bind to the Fab antibody domain and HRP can catalyse a chemilumeniscent signal.

There are a limited number of examples of biology influencing the design of lasers. Lasers use stimulated emission to emit spatially coherent, monochromatic beams of electromagnetic radiation with narrow divergence. The first laser was the ruby dye laser but since then many types of laser have been developed that are used in a vast number of different applications. For instance, random lasers use multiple scattering to provide optical feedback instead of mirrors. As biological tissues are strong scatterers, it has been possible to demonstrate laser action in dye infused biological tissues. In FRET lasers, the donor is excited and energy is transferred to the acceptor for lasing. A FRET laser has been built using DNA scaffolds to separate the donor and acceptor dyes and lasing was achieved at lower acceptor concentrations than conventional FRET lasers.

To date, biology has not been used to tackle the limitations of dye lasers. Common to all dye lasers is the use of a dye as the gain medium. The dyes are typically organic molecules and examples include rhodamine and fluorescein. One of their main advantages is their availability in a wide range of emission wavelengths. Their broad gain bandwidth is useful for generating short pulses via mode locking and gives them a broad wavelength tunability. Dye lasers might be used if a situation demands a readily tunable laser source or requires either high average powers or high pulse energies.

In most cases, the dye is dissolved in a solvent and a jet sprays die dye through the beam path so that only fresh dye is used. On safety grounds, it would be preferable for the operator not to work with solvents. Although solid state dye lasers have been constructed, the construction of an electrically pumped solid state dye laser system remains an active area of research. Dye lasers have developed a reputation for not being "user friendly" and their application is restricted to a limited number of situations. They are now less common than solid state lasers, which are made from crystals or glasses doped with transition or rare earth metals or semiconductors. Solid state lasers are more stable than dye lasers and can sometimes be electrically pumped.

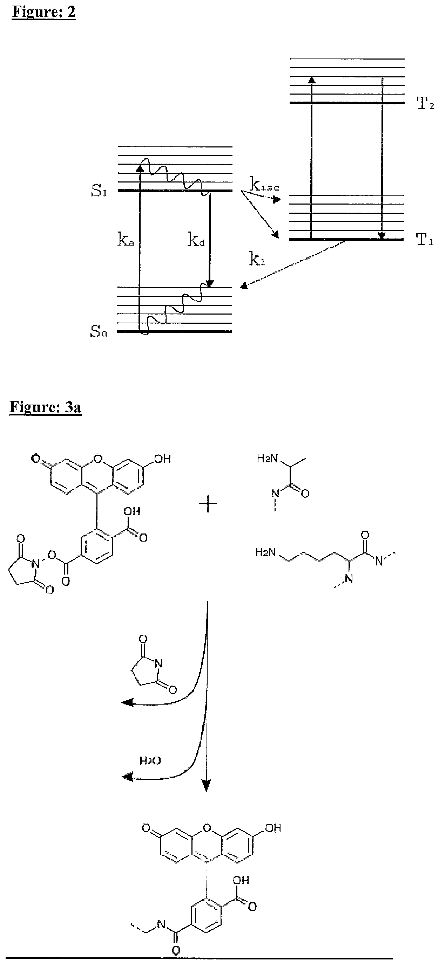

The most important limitations of laser dyes are related to the excited triplet state of the dye. Under pumping, there is a probability that electrons in the excited singlet state will make the spin forbidden transition to the excited triplet state, as shown in Figure 2. Electrons become trapped in the triplet state because relaxation to the ground state is spin forbidden. Over time, fluorescence is quenched because the population of electrons in the triplet state increases until a steady state is reached. Not only does the excited singlet state become depleted but the excited triplet state can often absorb the fluorescent emission. After a short period of time, losses are likely to exceed gain in dye gain mediums, preventing a sustained population inversion. On a longer time scale, dye in solution can be irreversibly bleached over time because the excited triplet state fluorescein molecules can react with other dye molecules and molecular oxygen to form non-fluorescent transient products. These transient products can act as intermediates towards permanently non-fluorescent products.

Kai Li et al. "Chemical Modification of M13 Bacteriophage and Its Application in Cancer Cell Imaging", Bioconjugate Chemistry, vol. 21, no. 7, 21 July 2010, pages 1369-1377 explains that the M13 bacteriophage has been demonstrated to be a robust scaffold for bioanomaterial development. Kai Li *et al.* goes on to describe how that they were able were able to conjugate small fluorescent molecules and cancer cell targeting motifs to an M13 bacteriophage to demonstrate potential applications of the M13 bacteriophage in bioimaging and drug delivery.

US 2010/0190233 A1 also discloses methods of modifying M13 bacteriophages. Similar to Kai Li et al., US 2010/0190233 A1 discloses dual-modified M13 bacteriophages were both cancer-targeting and fluorescent groups are covalently bonded to the M13 bacteriophages. US 2010/0190233 A1 explains that these modified M13 bacteriophages can not only be employed as a fluorescent probe in cancer imaging, but also can be used as biomaterials for cell alignment and scaffolding.

David L. Jaye et al. "Direct fluorochrome labeling of phage display library clones for studying binding specificities: applications in flow cytometry and fluorescence microscopy", Journal of Immunological Methods, vol. 295, no. 1-2, 1 December 2004, page 119-127 discloses a method of directly labelling phage clones with two fluorochromes. David L. Jaye *et al.* shows that the binding selectivity of peptides is maintained in flow cytometric analysis and immunofluorescence microscopy.

Kimberly A. Kelly et al. "In vivo imaging of molecularly targeted phage", Neoplasia, vol. 8, no. 12, 1 December 2006, pages 1011-1018 describes how a phage labelled with fluorochromes retain target specificity. Furthermore, Kimberly A. Kelly *et al.* explains that *in vivo* districution of the labelled phage can be quantified throughout the entire depeth of a tumour using fluorescent tomographic imaging. Finally, Kimberly A. Kelly *et al.* explains that the fluorescently labelled phage can serve as a replenishable molecular imaging agent.

WO 2005/034997 A2 discloses a composition comprising a conjugate of a photsensitiser and a bacteriophage. WO 2005/034997 A2 explains that the conjugate may be used to kill bacteria in a targeted method of photodynamic therapy..

Mariana Piuri et al. "Fluoromycobacteriophages for Rapid, Specific, and Sensitive Antibiotic Susceptibility Testing of Mycobacterium tuberculosis" PLOS ONE, vol. 4, no. 3, 20 March 2009, pages e4870-1 discloses a virus-based assay in which fluoromycobacteriophages are used to deliver a GFP or ZsYellow fluorescent marker gene to *M*. *tuberculosis.*

US 2011/0266470 A1 discloses apparatus, methods and systems for generating optical radiation, particularly for generating stimulated optical radiation from a biological gain medium, such as fluorescent proteins.

US 6141367 discloses a solid state dye laser comprises a gain medium which is in a substantially solid state and is doped with a fluorescent dye.

All of the above notwithstanding, organic dyes remain the molecules of choice for biological imaging, where fluorescent labels are necessary for the confocal microscopy of biomolecules and some biosensors and immunobiological assays. Inorganic quantum dot labels are making inroads, but they must be packaged to mitigate against toxicity, and subsequently functionalized to attach to the sites of interest, yielding moieties much more unwieldy than organic dye molecules. In particular, derivatives of dyes are available which allow the dye to be conjugated to the functional groups on proteins. Nonetheless, the photobleaching of dyes in confocal microscopy can prove problematic in some cases.

Accordingly, there is clearly a need to provide improved light-emitting compositions, which include lasing compositions and fluorescent compositions, which are programmable and which can therefore be used in a range of different biological and physical applications, such as in laser dyes and lasers, and in biological imaging applications, such as assays.

The inventors have developed new types of lasing compositions and fluorescing compounds, which are collectively referred to herein as a "fluorophage", and which have a wide range of useful applications. The fluorophage compositions described herein comprise an array of either dye molecules or light-emitting labels, which are scaffolded onto a programmable biological substrate.

Thus, according to a first aspect, there is provided a lasing composition comprising a proteinaceous biological substrate, wherein the biological substrate is chemically modified at specific attachment sites with light-emitting labels due to the presence of covalent bonds between the light-emitting labels and functional groups of amino acids present in the biological substrate, wherein the light-emitting labels comprise a fluorophore, the average molecular diameter of the light-emitting labels is between 0.5 nm and 2 nm, and the average distance between adjacent light-emitting labels is between 1 nm and 15 nm.

It will be appreciated that a lasing composition is one which is capable of acting as the source of optical gain in a laser. The distance between adjacent light-emitting labels on the biological substrate is preferably such that they are unable to chemically react with each other, but can still allow dipole-dipole interactions to occur between adjacent light-emitting labels.

In some embodiments, compositions of the invention may fluoresce.

Hence, the application also discloses a fluorescent composition comprising a biological substrate and an array of spaced apart dye molecules attached thereto, wherein the distance between adjacent dye molecules is such that they are unable to chemically react with each other, but can allow dipole-dipole interactions to occur between adjacent dye molecules.

It will be appreciated that a fluorescent composition is one which is capable of spontaneous emission of a photon resulting from a transition from an excited singlet state to the ground singlet state.

It is preferred that, following attachment to the substrate, the light-emitting labels in the lasing composition of the first aspect, and the dye molecules in the fluorescent composition of the second aspect, are unable to chemically react with each other, preferably when excited. If the light-emitting labels or dye molecules are too close together they quickly photobleach because they can chemically react with each other. Advantageously, as described in the Examples, the inventors have demonstrated that the compositions of the invention have a significantly reduced photobleaching rate compared to free light-emitting labels or dye molecules in solution, because the light-emitting labels or dye molecules are too far apart from each other for them to exchange electrons.

It is also preferred that, following attachment, the distance between adjacent light-emitting labels and dye molecules is such that dipole-dipole interactions can occur between adjacent light-emitting labels or dye molecules. Such dipole-dipole interactions are important as they provide alternative deactivation pathways for the molecules (e.g. light-emitters or dyes) to return to the ground state from the excited triplet state. It will be appreciated that the triplet state quenches lasing and can also result in photobleaching. Advantageously, as described in the Examples, the triplet state lifetime of the compositions of the invention is dramatically reduced because of the high number of neighbouring light-emitters or dye molecules sufficiently within range of each other for sufficient dipolar interactions to occur, which enhances triplet state quenching. Thus, the compositions of the invention are much more stable, and significantly minimise photobleaching.

The inventors have shown that the light-emitting labels or dye molecules can be scaffolded with nanoscale precision onto the surface of the biological substrate, as they will only covalently bond to particular chemical groups (i.e. the attachment sites). Since biological substrates are programmable, the chemical nature of the substrate can be readily modified by making either genetic mutations or post-translational modifications thereto. Consequently, the position of the light-emitting labels or dye molecules on the substrate can be "programmed" to form arrays with different optical properties. As the substrate is programmable, it is also possible to introduce genetic modifications that cause protein fusions to be displayed on the surface of either the lasing composition of the first aspect, or the fluorescent composition of the second aspect. These protein fusions may be used to bind the biological substrate to surfaces, or other biomolecules, or non-biological nanoparticles. In one embodiment, the substrate may comprise (or be modified to comprise) one or more cysteine residues each with an exposed thiol group. The thiol group may be positioned so that it can act as a reducing agent towards the scaffolded dye molecules. For example, 2-Mercaptoethylamine (MEA) is effective at reducing the photobleaching rate of the dye, fluorescein. The thiol group on MEA is responsible for its properties as a reducing agent. Therefore, adding a plurality of thiol groups to the surface of the substrate helps to reduce photobleaching.

Preferably, the light-emitting labels or dye molecules are attached to specific, spaced-apart attachment sites, which are disposed along the structure of the biological substrate. These potential attachment sites are preferably regularly spaced apart (i.e. ordered) along the substrate. In other words, the distance between adjacent potential attachment sites may be substantially the same along the substrate. The positions of the potential attachment sites may create a repeating pattern along the substrate. Preferably, the potential attachment sites are not randomly arranged along the substrate. The light-emitting labels or dye molecules may not self-assemble to form a monolayer on the substrate.

It will be appreciated that whether or not interactions occur between the light-emitting labels or dye molecules on the surface of the biological substrate depends on several factors, including the bulkiness of the light-emitter or dye molecule itself, the separation of the attachment sites along the structure of the biological substrate and the rotational freedom of the light-emitters or dye molecules, as well as the intrinsic electronic properties of the light-emitters and the substrate, or the dyes and the substrate. Thus, what constitutes as being too close and too far spacing along the substrate depends on the size of the light-emitting label or dye molecule.

The inventors believe that dipole-dipole interactions may be weak between light-emitting labels or dye molecules with an intermolecular separation greater than 10nm or 15nm. However, if the separation is less than 1nm, then the light-emitting labels or dye molecules may chemically react unless otherwise hindered. Therefore, in some embodiments, the average distance between adjacent light-emitting labels or dye molecules may be between 1nm and 10nm, or between 1nm and 7nm, or between 1nm and 5nm, or between 1nm and 3nm. In other embodiments, the average distance between adjacent light-emitting labels or dye molecules may be between 2nm and 15nm, or between 2nm and 10nm, or between 2nm and 7nm, or between 2nm and 5nm, or between 2nm and 3nm.

The light-emitting labels or dye molecules may comprise organic or inorganic light-emitters or dyes. However, organic light-emitting labels or dyes are preferred. Thus, the light-emitting labels or dye molecules may comprise hydrocarbons or hydrocarbon derivatives that possess at least one conjugated double bond. The light-emitting labels or dye molecules preferably absorb wavelengths between 220 nm and 1000 µm. For example, the light-emitting labels or dye molecules may absorb in the visible (i.e. 390-750 nm), the ultraviolet (i.e. 10-400 nm) or the infrared (1m-750nm) parts of the spectrum. Thus, the light-emitting labels or the dye molecules may be capable of absorbing between 240 nm and 900 nm, or between 460nm and 500nm, or between 900nm and 3000nm, or between 3-8 µm (mid-infrared), or between 8-15 µm (long-infrared), or between 15-1000 µm (far-infrared).

Suitable light-emitting labels used in the compositions of the invention may be characterised by their capability to emit light via a transition from a higher quantum state to a lower quantum state and a functional group that allows the labels to be covalently attached to a biomolecule without compromising its light emitting capability. Suitable dye molecules used in the compositions of the invention may comprise hydrocarbons or derivatives thereof, which possess at least one conjugated double bond.

The light-emitting labels or dye molecules may be members of the xanthene family of dyes, or other optical moieties or light-emitters, such as GFP or quantum dots. The dye molecules comprise Rhodamine or a derivative thereof, such as Rhodamine B, Rhodamine 6G or Rhodamine 123.

As described in the Examples, the light-emitting labels or dye molecules may comprise fluorescein or a derivative thereof. It will be appreciated that fluorescein has many derivatives, including fluorescein isothiocyanate (FITC), NHS-fluorescein or 6-FAM phosphoramidite.

In one embodiment, the lasing composition or fluorescent composition may comprise one type or species of light-emitting label or dye molecule. However, in another embodiment, more than one type or species of light-emitting labels or dye molecules may be scaffolded to the substrate. For example, the composition may comprise one or more type of light-emitting labels or dye molecules from the xanthene family (e.g. fluorescein and Rhodamine, etc.).

The biological substrate may be any surface comprising a biomolecule, which is subjectable to chemical modification for the attachment of the dye molecules to potential attachment sites. The biological substrate may not comprise carbohydrate or lipid. The biological substrate is preferably self-assembled into a well-defined, regular structure. For optimum use in the lasing and fluorescent compositions of the invention, the biological substrate preferably comprises chemical attachment sites that form a regular array therealong. The biological substrate may comprise a virus or virus-like particle.

The amino acid residues to which the light-emitting labels or dye molecules are attached may be either natural (i.e. the 20 amino acids which form naturally occurring peptides), or non-natural. The substrate may be substantially elongate, or rod-like. For example, the substrate may be at least 500Å, 1000Å, 2000Å, 3000Å, 4000Å, 5000Å, 6000Å, 7000Å, or 8000Å in length.

The biological substrate may comprise a wild-type or mutant biological substrate, including a bacteriophage, actin fiber, biomimetic compound, or other proteinaceous substrate. The substrate may comprise an RNA-protein complex, which may be expressed in *E. coli.*

Bacteriophages and viruses, which may be used as the substrate may be filamentous or non-filamentous. The virus may be a plant virus, for example cowpea mosaic virus (CPMV) and tobacco mosaic virus (TMV), as described in the Examples. The TMV may be expressed in *E. coli.*

As described in the Examples, the biological substrate may comprise M13 filamentous bacteriophage (M13). Thus, in a preferred embodiment, the fluorescent composition may comprise fluorescein dye molecules scaffolded in ordered arrays on M13 bacteriophage substrate. This class of composition or "fluorophage" has been termed herein as "M13-fluorophage". The fluorescein derivative dye molecules react specifically to the primary amine groups on the N-terminus and lysine residues on the major coat protein of M13. The fluorescein molecules may be spaced apart by 1 nm to 3 nm, and by at least 2 nm, which the inventors have shown is a distance which prevents electron transfer between adjacent light-emitting labels or dye molecules, thereby preventing photobleaching. However, dipolar interactions between the dye molecules are still possible, thereby providing alternative deactivation pathways for the dyes to return to the ground state from the excited triplet state.

The effective concentration of the fluorescein derivatives on the surface of M13 is greater than that for the same number of dyes in solution, and so the rate of triplet-triplet annihilation is dramatically increased. Consequently, the excited triplet state lifetime is reduced. Additionally, if the sample is flushed with argon or nitrogen to remove molecular oxygen, then the formation of reduced and semi-oxidized form of the dye would be prevented. Advantageously, by reducing the population of electrons in the excited triplet state, the biological scaffold can be used to make dye lasers with longer pulse lengths and faster repetition rates and lower rates of photobleaching. Therefore, the inventors believe that the application of programmable biological substrates in the composition of the first aspect represents a new paradigm in laser technology.

Thus, in a second aspect, there is provided use of the composition of the first aspect as a laser dye.

In a third aspect, there is provided a laser dye comprising the composition of the first aspect.

The lasing composition of the invention may act as a gain medium in the laser dye, or as a saturable absorber. Thus, it will be appreciated that the laser dye acts as a lasing medium. Advantageously, the use of ordered, regularly-spaced, genetically programmable attachment sites on the biological substrate (e.g. a rod-like virus or phage) in the lasing or fluorescent composition of the invention allows the laser dye of the fourth aspect to exhibit improved lasing properties and decreased dye degradation, when compared to the use of an amorphous substrate in which the attachment sites are randomly located thereon. As discussed above, this is because the regular spacing of the attachment sites and thus light-emitting molecules or dye molecules along the substrate ensures that the distance between adjacent molecules is such that they are unable to chemically react with each other, but allow dipole-dipole interactions to occur therebetween, thereby avoiding photobleaching.

In fourth aspect, there is provided use of the composition of the first aspect as a saturable absorber.

It will be appreciated that the composition therefore acts as a medium for electromagnetically-induced transparency.

The laser dye may be used in a laser, for example a dye laser.

Thus, in a fifth aspect, there is provided a laser comprising the laser dye of the third aspect.

The laser may be selected from the group of lasers consisting of: a plasmonic laser; a single-particle plasmonic laser; a solid state laser; an electrically pumped solid state laser; a dye laser; a liquid dye laser; a tunable laser; a pulse laser; and an ultrashort pulse laser. The laser may be used in laser treatment targeted to a specific cell type.

The laser may be a dye laser. A dye laser may comprise the dye mixed with a solvent, which may be circulated through a dye cell, or streamed through open air using a dye jet. The laser may comprise a high energy source of light to "pump" the liquid dye beyond its lasing threshold. The high energy source may comprise a fast discharge flashlamp or an external laser. The laser may comprise one or more mirrors to oscillate the light produced by the dye's fluorescence, which is amplified with each pass through the liquid dye. An output mirror is normally around 80% reflective, while all other mirrors are usually more than 99% reflective. Alternatively, metallic nanostructures adjacent to or attached to the biological scaffold could provide feedback through surface plasmon resonance. The dye solution may be circulated at high speeds, to help avoid triplet absorption and to decrease degradation of the dye. A prism or diffraction grating may be mounted in the beam path, to allow tuning of the beam.

The inventors have shown that the dye lasers of the invention have several significant advantages over conventional dye lasers. Firstly, the laser dye comprising the fluorescent composition of the first aspect does not photobleach as quickly as conventional dye molecules, and so they do not need to be pumped through the optical cavity. Secondly, the dye could be cast into viral films or blocks to make a solid state gain medium. Thirdly, tunable dye lasers are able to sustain longer pulse lengths than conventional dye lasers. Fourthly, the lasers of the invention overcome the limitations of the previous generation of dye lasers because they do not require dye to be pumped through the optical cavity. Fifthly, mirrorless lasing is achievable with the fluorescent composition of the first aspect at lower pump powers than is possible with free dye molecules in solution.

The fluorescent composition of the invention may be used to construct solid state or liquid dye lasers with protein fusions that enable electrical, optical and chemical reactions to pump energy into the laser. Also, aside from their scaffolding properties, the M13 phage forms viral cast films, and this property can be readily exploited to build a solid state dye laser. Additionally, the fluorescent composition of the invention may be cast onto an organic polymer film that conducts electricity so that there is near field energy transfer between the organic polymer and the fluorophage film, and such a system would be capable of electrically pumped laser action. A pump laser would act as a source by pumping the fluorescent composition (e.g. a single fluorophage) to just under its threshold power. A probe laser would act as a gate by causing the single pumped fluorophage to emit stimulated emission. The emission could be collected at the drain. The laser may therefore be a polymer laser. The fluorescent composition may be a dye doped in a polymer matrix of a polymer laser.

The composition may be used in a wide range of biomedical applications, for example a protein microarray. For example, the inventors have also shown that the lasing composition of the first aspect and the fluorescent composition of the second aspect may be effectively used in biosensors and in biological assays, for detecting small molecules.

Therefore, in a sixth aspect, there is provided use of the composition of the first aspect as a biosensor, or in biological imaging applications.

Therefore, in a seventh aspect, there is provided a biosensor or biolabel comprising the composition of the first aspect.

The biosensor may be a protein microarray biosensor. The composition may be used in an assay. The composition may be fused to a protein or other biological entity or small molecule, or substrate. Thus, the fluorescent composition may comprise a fusion protein attached thereto. As described in the Examples, in one embodiment, the g3p coat protein of the M13-fluorophage may be genetically engineered to display the BB-domain from protein A. The BB-M13 fluorophage may bind IgG antibodies and be used in the same way as secondary antibodies to detect proteins in biological assays, including Western blots and ELISA. The main advantage of this would be greater sensitivity to lower protein concentrations and a more quantifiable, repeatable emission compared to using the chemiluminescence from an enzyme-based secondary antibody system.

It will be appreciated that in a conventional assay, the fluorophore emits light as spontaneous emission in all directions. However, in an assay involving the use of the fluorescent composition of the invention, the composition can emit light as stimulated emission in one preferred direction. This is possible because the composition is more resistant to immediate photobleaching. Advantages of using stimulated emission in this manner are a superior signal to noise ratio, and a narrower bandwidth emission.

The inventors have found that the fluorescent composition may be assembled into larger structures, including liquid crystals and films.

Thus, in an eighth aspect, there is provided a liquid crystal structure or film comprising the composition of the first aspect.

Furthermore, in a ninth aspect, there is provided use of the liquid crystal structure or film according to the eighth aspect, as a display.

In eleventh tenth aspect, there is provided a display comprising the composition of the first aspect.

For example, the display may be a display for any computation, control or communications devices (e.g. a mobile phone). Advantageously, the display is more energy efficient because stimulated emission can be used instead of spontaneous emission.

Furthermore, the composition may be used as a component in an optical computer.

Thus, in an eleventh aspect, there is provided an optical computer comprising the composition of the first aspect.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying diagrammatic drawings, in which:-
**Figure 1a** illustrates the M13 major coat protein assembly, and **Figure 1b** illustrates the structure of the M13 major protein;
**Figure 2** illustrates the eigenstates of fluorescein. Radiative and non-radiative transitions are shown in solid and dash lines, respectively. Internal conversion is represented by the sinusoidal lines;
**Figure 3** shows the chemical modification of wild-type M13 to form M13-fluorophage. **Figure 3a** shows NHS-fluorescein attached via an amide bond to the N-terminus or to the functional group of a lysine residue. **Figure 3b** shows a model of a reaction between NHS-fluorescein and the amines exposed at the surface of M13. Residues with an amine are shown in bold. **Figure 3c** is a schematic drawing detailing the position of addressable chemical linkage sites on the surface of M13;
**Figure 4** shows FLIM decay curves;
**Figure 5a** shows fluorescence spectra, and **Figure 5b** shows fluorescence over time under continuous pumping: only a fraction of the emitted fluorescence is allowed to reach the detector so that it does not become saturated; as such, only the relative change in intensity is important. Con. Dye = 1mg/ml; diluted dye = 10µg/ml;
**Figure 6** is a schematic diagram of a tunable dye laser setup with a prism and fluorophage cuvette; and
**Figure 7** is a schematic diagram of a passive mode-locked continuous wave dye laser setup with fluorophage cuvette, acting as a gain medium;
**Figure 8** shows the use of BB-fluorophage as a secondary antibody;
**Figure 9** shows UV-Vis absorption spectrum used in fluorophage threshold experiments;
**Figure 10** is a schematic diagram of a laser setup for conducting threshold measurements;
**Figure 11** is a threshold curve for M13-fluorophage according to the invention;
**Figure 12** is a schematic representation of a genetic construct known as Tobacco Mosaic Virus (TMV) coat protein (CP) with C27A and C127C mutations;
**Figure 13** shows the structure of the TMV coat protein (TMVCP) showing the C27A and M127C mutations, and RNA packaged by the coat protein. Crystal structures were taken from protein data bank 20M3;
**Figure 14** is a schematic representation of a genetic construct for a TMV RNA transcript;
**Figure 15** shows the structure of the helical rods made when the protein monomers from Figure 12 self-assemble to package the RNA transcript. The view is from looking down the rod;
**Figures 16-18** are transmission electron micrographs of recombinant TMV (rTMV). The rTMV molecules can be easily identified as the ring structures shown in each Figure; and **Figure 19** shows reaction schemes used for the modification of rTMV with dyes.

### Examples

As described in the Examples below, the inventors have developed a composition (referred to herein as a "fluorophage") in which a light-emitting label or dye molecule (e.g. fluorescein) is conjugated to a programmable biological substrate (e.g. M13 bacteriophage, or Tobacco Mosaic Virus). The light-emitting labels or dye molecules are regularly spaced-apart to form an array on the biological substrate, which thereby acts as a scaffold structure holding the dye molecules in position. The inventors have analysed the light-emitting and/or fluorescence characteristics of die fluorophage, and have used it as a dye in a dye laser, and instead of molecular dyes for biosensing and biological imaging applications. The lasing properties of die fluorophage make an excellent candidate as a light-emitting composition in a wide range of applications.

### Example 1 - Preparation of an M13-fluorophage

The dye used was 5-carboxyfluorescein, succinimidyl ester (5-FAM, SE) powder (Invitrogen), Herein, the term "dye" and "light-emitting label" are used interchangeably. It was dissolved in spectroscopic grade dimethyl sulfoxide (DMSO) (VWR) to a final concentration of 10 mg/ml. Referring to Figures 1a and 1b, there is shown the structure of the M13 major coat protein assembly. M13 was therefore propagated in a 400 ml culture of *E*. *coli* Top10f' in Terrific Broth at 37°C with shaking. The M13 was then purified using standard methods to a final titer of 2.2 ± 0.9 x 10¹³ pfu/ml. All buffers were autoclaved and filtered sterilized to remove any contaminants larger than 0.22 µm.

19 µl of the 5-FAM, SE stock dye was added to 100 µl of the M13 phage stock and left for one hour at 37°C with shaking in the dark to form the M13-fluorophage. The reaction was conducted at pH 7.5 to encourage single labelling of the coat proteins. The reaction was quenched with 1 M TRIS pH 8.5. The reaction was subjected to centrifugation to remove any insoluble reaction products or polymerised dye. The sample was then precipitated using 5% PEG-8000/200 mM NaCl and resuspended. The sample was subjected to further rounds of precipitation and resuspension until the supernatant was colourless by sight. The sample was then buffer-exchanged using a Zeba desalt spin column (Thermo Scientific) and subjected to a final precipitation with 5% PEG-8000/200 mM NaCl to concentrate the sample.

The M13-fluorophage samples and free 5-FAM, SE molecular dye samples were loaded into square capillaries with widdis of 0.7 mm by capillary action. The fluorophage sample was shaken to move the sample into the middle of the capillary. The loaded capillaries were then left in the dark at 4°C.

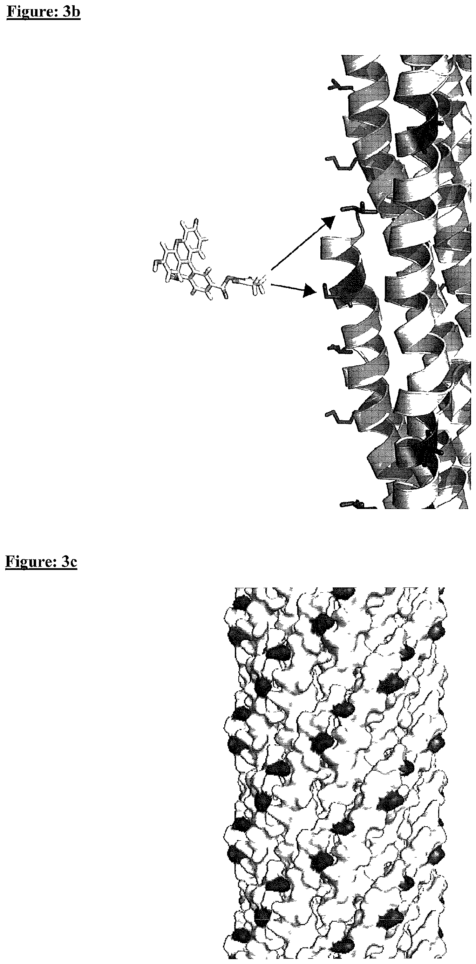

Referring to Figures 3a-3c, there are shown the attachment of fluorescein (i.e. NHS-fluorescein) via an amide bond to the N-terminus or the functional group of a lysine residue. NHS-fluorescein is a N-Hydroxysuccinimide ester labelling reagent and can form stable amide bonds with primary amines, releasing N-hydroxysuccinimide as shown in 3a. There are two surface exposed primary amines on the surface of M13 as shown in 3b. These can be selectively modified because the amines on the N-terminus and lysine functional group have different pKₐ values. An array of dye molecules is formed because the chemically addressable sites form a well ordered array as shown in 3c.

### Example 2 - Fluorescence lifetime imaging microscopy (FLIM) using M13-fluorophage

Fluorescence lifetime imaging microscopy (FLIM) was conducted on a Zeiss Axioskop META 510 NLO microscope with a Becker and Hickl FLIM system to test whether the M13-fluorophage prepared in Example 1 has decreased triplet state lifetimes and decreased steady state triplet state populations compared to free dye molecules in solution.

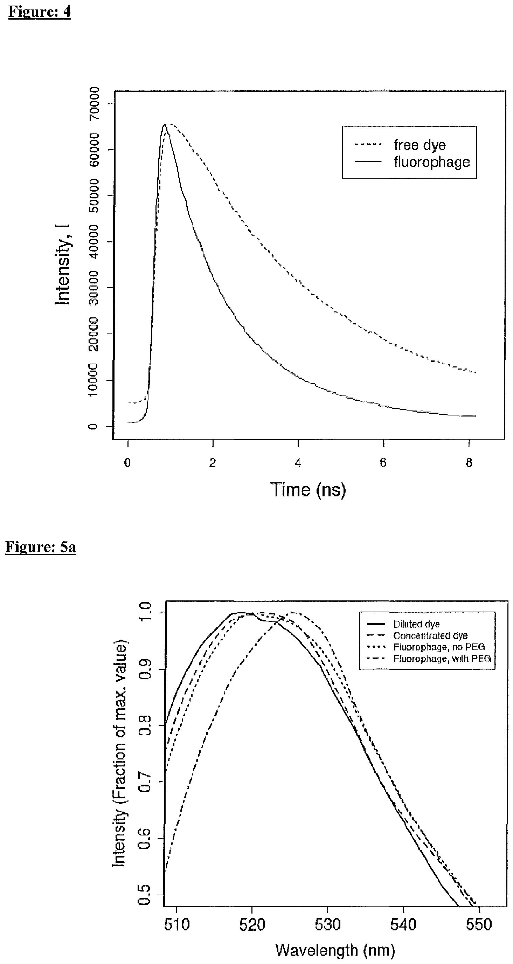

Referring to Figure 4, there is shown FLIM decay curves of the free dye (5-FAM, SE) and the M13-fluorophage prepared in Example 1. As can be seen, there is one component to the fluorescence lifetime of free fluorescein dye molecules of approximately 3 ns but there were two approximately equal components to the fluorescence lifetime of M13-fluorophage of approximately 1 ns and approximately 2 ns. It will there be appreciated that the M13-fluorophage decays faster, and therefore has more deactivation pathways. This reduction in the lifetime for M13-fluorophage is due to die dipolar interactions between die scaffolded dyes, which open up more deactivation pathways.

In summary, this experiment measures the time taken for electrons in die excited states to return to the ground state. The shorter the time, die more deactivation pathways are available for electrons to return to the ground state. On the fluorophage of the invention there are more deactivation pathways because of the dipole-dipole interactions between neighbouring dyes. These dipole-dipole interactions are important for quenching the triplet state, and these experiments prove that they are there.

### Example 3 - Confocal microscopy using M13-fluorophage

Fluorescence spectra and fluorescence time series were acquired using an Olympus Confocal Inverted Laser Scanning Microscope with a 10x objective lens to test whether the M13-fluorophages prepared in Example 1 have decreased photobleaching rates compared to free dye molecules in solution.

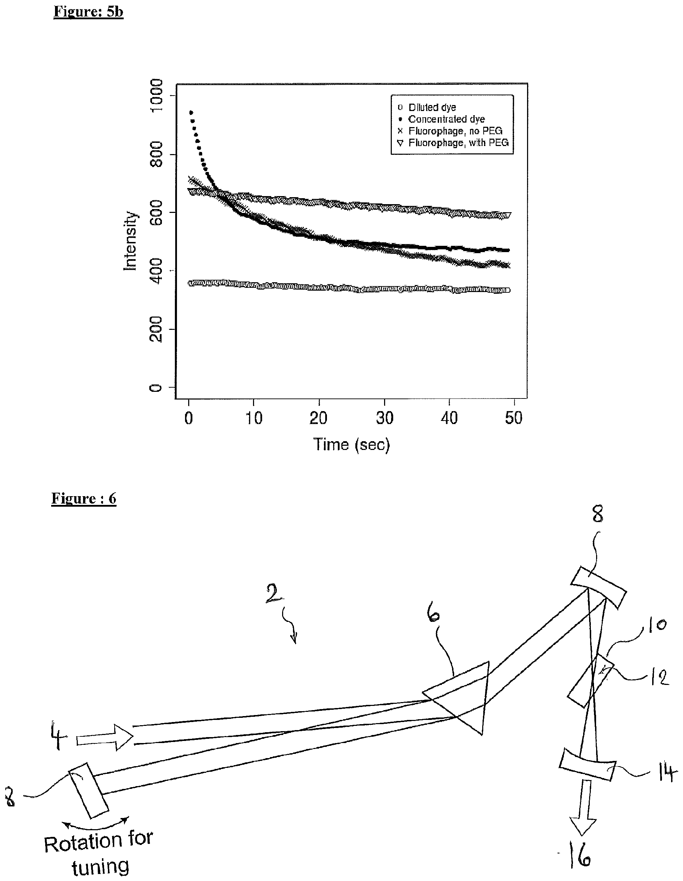

Referring to Figure 5, there is shown fluorescence spectra (Figure 5a), and fluorescence over time under continuous pumping (Figure 5b). For each sample, the fluorescence spectra were measured. All of the samples were pumped with a 25 mW laser set at 40% power except die concentrated dye sample which was pumped with the same laser set at 1% power. As can be seen, the fluorescence peak of the concentrated free molecular dye solution (5-FAM, SE) and both of the M13-fluorophage samples were red-shifted compared to the peak of the diluted solution. The most red shifted fluorescence peak was the M13-fluorophage sample with 5% PEG-8000/200 mM NaCl. For each sample, die intensity of the fluorescence over time under continuous pumping with a 25 mW laser set at 40% power was measured. The mean separation of the dye molecules is significantly greater for die diluted dye sample than for the concentrated dye sample (see Table 1). The number of dyes per phage is an estimate, and is included to illustrate that the density of dyes on die surface of die phage is closer to that of the concentrated dye than the diluted dye. Consequendy, collisions between dye molecules in die excited triplet state are much rarer for the diluted dye sample, which causes a reduction in the rate of photobleaching.

**Table: 1 - Concentration and mean separation of dye molecules**

| | Concentration | Mean separation |
|---|---|---|
| Concentrated dye | 1 mg/ml | 9.2 nm |
| Diluted dye | 10 µg/ml | 42.5 nm |
| M13-fluorophage | 10³ dyes per phage | 4.5 nm |

As shown in Figure 5, the diluted molecular dye sample and the M13-fluorophage sample with 5% PEG-8000/200 mM NaCl underwent a comparable rate of photobleaching. However, the mean separation of the diluted dye molecules was approximately ten times greater than that of the dye molecules scaffolded to the surface of M13, For the M13-fluorophage, collisions between dye molecules in the excited triplet state are rare because the dyes are scaffolded. For the diluted dye sample, collisions are rare because the mean separation of the dye molecules is so great.

The M13-fluorophage sample without 5% PEG-8000/200 mM NaCl had significantly lower rates of photobleaching than the concentrated molecular dye sample despite having dye molecules scaffolded to a comparable mean separation. This indicates that the dye molecules on the surface of the M13-fluorophage were not able to exchange electrons with neighbouring dye molecules.

The rate of photobleaching is reduced for M13-fluorophage samples containing 5% PEG-8000/200 mM NaCl. PEG-8000 is a molecular crowding agent that causes die local concentration of M13-fluorophage to increase. M13 forms liquid crystals at high concentrations so it is reasonable to anticipate that M13-fluorophage is also capable of forming crystal structures. The hydrogen bonding network between M13-fluorophage in a liquid crystal is likely to prevent fluorophage-fluorophage collisions, which explains why M13-fluorophage in solutions with 5% PEG-8000/200 mM NaCl undergo an even greater reduction in their rate of photobleaching.

Over time, the intensity of the fluorescence would tend towards zero if die dyes were unable to diffuse away from the laser path. However, as the dyes can diffuse away from the laser path, the intensity reaches an asymptote where the rate of fresh dye diffusing into the path of the laser matches the rate of photobleached dye out of the path. This effect is more pronounced for the molecular dyes because they are much smaller than M13-fluorophage and so can more readily diffuse out of the path of the laser beam.

### Example 4 - Use of fluorophage in dye lasers

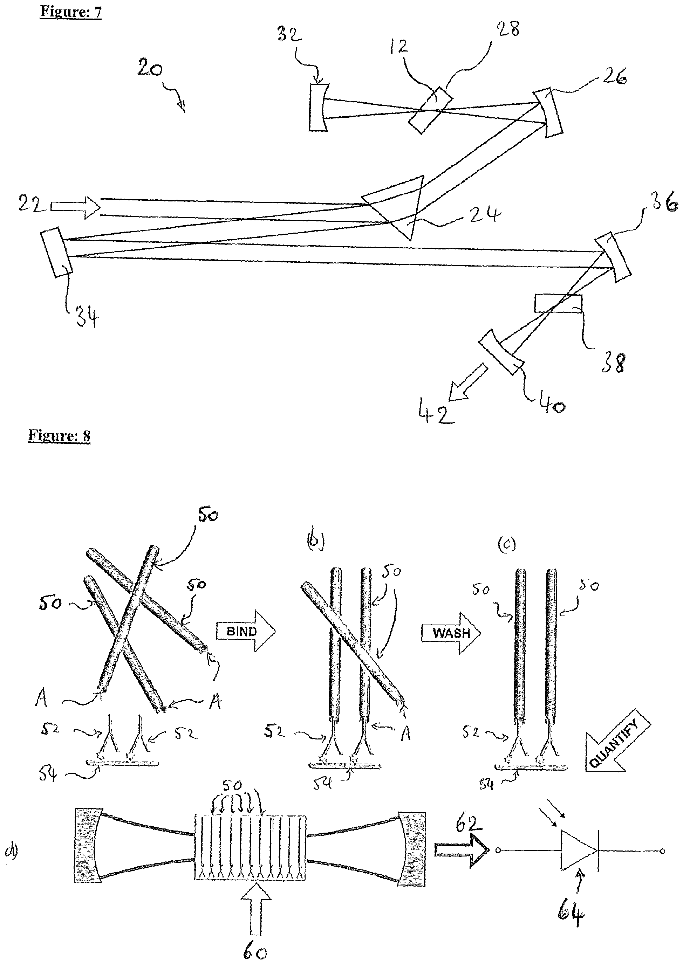

Based on the data produced from Examples 2 and 3, the inventors have shown that the fluorophages of the invention, as prepared by Example 1, can be readily used instead of molecular dyes in dye laser systems. Two types of dye lasers with commercial value are tunable lasers and ultrashort pulse lasers, which are illustrated in Figures 6 and 7, respectively. The inventors have shown that a dye cell containing the fluorophage of the invention can be placed in the beam path of either laser system, which benefit from a number of advantages, as discussed below.

### (i) Tunable lasers

Tunable dye lasers can emit at a broad range of wavelengths, and an example of such a laser 2 is shown in Figure 6. The fluorophage 12 is pumped by an excitation beam 4 of the appropriate wavelength. The excitation beam 4 is passed through a dispersive element, in this case a prism 6, so that die beam refracts towards a first mirror 8. The excitation beam is reflected towards a cell 10 containing the fluorophage 12. Emission from the fluorophage 12 is reflected at mirrors 14 and 8 towards the prism 6. The emission from the fluorophage 12 is at a different wavelength to the excitation beam so the angle of refraction from the prism 6 is different. Mirror 18 is rotated to tune the optical cavity so that losses for one particular wavelength are much smaller than for other wavelengths. These mirrors 8, 14, 18 and the prism 6 make up the optical cavity. Mirror 14 is not 100% reflective so output light 16 is transmitted through mirror 14.

Tunable lasers 2 are especially useful in applications where scattering is observed as a function of wavelength. Dye lasers can be used to make tunable ultraviolet and infrared lasers sources by frequency mixing in non-linear materials. Applications of tunable dye lasers, where the fluorophage dye cell shown in Figure 6, could be employed include:-
- Raman scattering experiments;
- Observing the resonant scattering from atoms and molecules;
- Atomic absorption and fluorescence spectroscopy;
- Optical pumping at wavelengths inaccessible to diode lasers;
- Photomagnetism experiments;
- Generation of tunable UV and IR laser light;
- Photochemistry;
- LIDAR; and
- Laser spectroscopy.

### (ii) Ultrasbort pulse lasers

With reference to Figure 7, picosecond and femtosecond lasers 20 are useful for making time resolved measurements. The fluorophage 12 is pumped by an excitation beam 22 of the appropriate wavelength. The excitation beam 22 is passed through a dispersive element, in this case a prism 24, so that the beam refracts towards a first mirror 26. The excitation beam is reflected towards a cell 28 containing the fluorophage 12. Emission from the fluorophage 12 is reflected at mirrors 32 and 26 towards the prism 24. The emission from the fluorophage 12 is at a different wavelength to the excitation beam so the angle of refraction from the prism 24 is different. The emission from the fluorophage is reflected at mirrors 34, 36 towards a saturable absorber 38. The saturable absorber is responsible for passively mode-locking the system. Mirror 40 makes up the optical cavity along with mirrors 32, 26, 34, 36 and the prism 24. Mirror 40 is not 100% reflective so output light 42 is transmitted through mirror 40.

Ultrashort pulse lasers 20 can be used to measure the relaxation times of atoms and molecules and to monitor chemical reactions. Applications of ultrashort pulse lasers where the fluorophage dye cell can be employed include:-
- Time resolved fluorescence;
- Non-linear spectroscopy; and
- Pump-probe experiments.

The inventors have shown that fluorophage dye lasers 2, 20 have a number of major advantages over conventional dye lasers, including:-
(i) The fluorophage dye does not photobleach as quickly as conventional dye molecules, so they do not need to be pumped through the optical cavity;
(ii) The fluorophage dye could be cast into viral films to make a solid state gain medium;
(iii) Tunable fluorophage dye lasers are able to sustain longer pulse lengths than conventional dye lasers.
(iv) The previous generation of dye lasers required die operator to pump toxic solvents around the lasers system, and so they developed a reputation for not being "user friendly".
The fluorophage lasers 2, 20 shown in Figures 6 and 7 overcome the limitations of the previous generation of dye lasers because they do not require dye to be pumped through the optical cavity.

The market for lasers is very broad because lasers have so many applications. Laser manufacturers, such as Coherent and Spectra-Physics (Newport Corp), are especially keen to acquire and develop technologies that give them an advantage over the competition.

Ti:Sapphire lasers are the industry standard for generating ultrashort pulses. However, even after frequency doubling, these lasers have wavelengths greater than 350 nm, which is too long to excite intrinsic fluorescence in proteins.

### Example 5 - Use of fluorophage in biosensors and in biological imaging

The fluorophage of the invention can also be used instead of molecular dyes in a range of biosensing and biological imaging applications. For example, protein fusions can be displayed at the surface of the fluorophage.

Referring to Figure 8, there is shown the use of the fluorophage in a biological assay. The g3p coat protein of the fluorophage has been genetically engineered to display the BB-domain from protein A of *Staphylococcus aureus* in a construct denoted as BB-M13 fluorophage 50. This BB-M13 fluorophage 50 then binds IgG antibodies 52 bound to a support surface 54 and is used in the same way as secondary antibodies to detect proteins in biological assays, including Western blots and ELISA. As shown in Figure 8a, a solution containing the fluorophage 50 is added to the antibodies 52 bound on the support surface 54 to allow binding, as shown in Figure 8b. The support 54 is then washed to remove unbound fluorophage 50, as shown in Figure 8c. Finally, the amount of bound fluorophage 50 can then be quantified based on the fluorescence of the fluorophage 50, as shown in Figure 8d. Importantly, the amount of bound fluorophage can be quantified by the laser emission of the fluorophage. For example, the fluorophage 50 can be excited by a laser 60, and laser emission 62 can be detected via a photodiode 64. This would not be possible usually because conventional dyes would be too sensitive to photobleaching. An assay based on laser emission would have a much superior signal to noise ratio. The main advantage of this method is the far greater sensitivity to lower protein concentrations and a more quantifiable, repeatable emission compared to using the chemiluminescence from an enzyme-based secondary antibody system.

In die case of a lasing fluorophage, this can offer an entirely new means of obtaining contrast in an image. Organic dyes remain the molecules of choice for biological imaging, where fluorescent labels are necessary for the confocal microscopy of biomolecules and some biosensors and immunobiological assays. Inorganic quantum dot labels are making inroads, but they must be packaged to mitigate against toxicity, and subsequently functionalized to attach to the sites of interest, yielding moieties much more unwieldy than organic dye molecules. In particular, derivatives of dyes are available which allow the dye to be conjugated to the functional groups on proteins. Nonetheless, the photobleaching of dyes in confocal microscopy can prove problematic in some cases.

The inventors have shown that the fluorophage of the invention can make a big impact in the biosensing and biological imaging fields. Advantages of fluorophage dyes over conventional dyes include: -
(i) The fluorophage is less susceptible to photobleaching than conventional dyes;
(ii) The fluorophage is brighter than single dye molecules because there are over a hundred (or thousand) molecular dyes attached to the biological substrate per fluorophage;
(iii) The fluorophage can be programmed to bind to biological and non-biological targets;
(iv) Unlike assays that use enzyme-conjugated secondary antibodies, the amount of fluorescent emission from a fluorophage does not depend on temperature, exposure time or other experimental variables.

Dyes are used ubiquitously as fluorescent labels for assays and confocal microscopy in bio labs worldwide. The fluorophage of the invention represents a better alternative to molecular dyes for many of these applications. Optical based assays are sold by many biotech companies, including Thermo Scientific and Merck. In addition, since a single fluorophage can act as a laser, because fluorophages are less susceptible to photobleaching, this would be revolutionary for biosensing and microscopy, since the signal to noise ratio would be greatly enhanced in comparison to the fluorescence of a single dye molecule.

### Example 6 - Demonstration of a fluorophage laser

Preliminary evidence that the compositions of the invention could sustain laser action was acquired. Fluorescein isothiocyanate was titrated into a M13 phage solution prepared as in the previous examples. Reactions were quenched with 1M pH 7 TRIS. Insoluble products were removed through two centrifugation steps. After the second centrifugation step, only a small pellet precipitated to the side of the tube. Fluorophage was separated from smaller molecular weight products by 5% PEG-8000/200 mM NaCl precipitation.

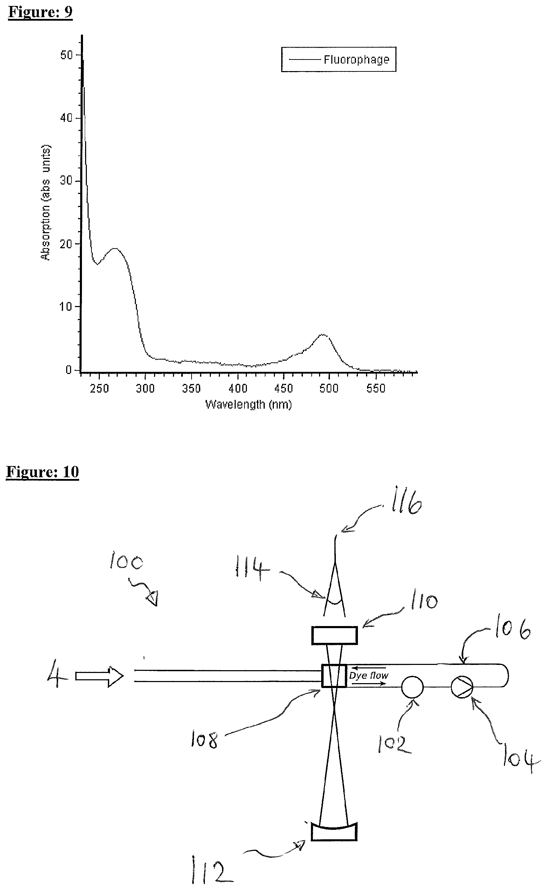

As shown in Figure 9, the UV-Vis absorption spectrum of the sample contains a characteristic fluorescein peak close to 490 nm (Digilab Hitachi U-1800 spectrophotometer). The ratio of the characteristic M13 phage peak at 269 nm and the fluorescein peak at 490 nm indicates that there were approximately 265 dyes per phage. The fluorophage solution had an optical density equivalent to a 23.7 µg/ml fluorescein solution.

A dye laser 100 was built to test whether the composition was capable of sustaining laser action, and this is shown in Figure 10. The fluorophage 12 is stored in a dye reservoir 102 and continuously pumped via peristaltic pump 104 through silicone tubing 106 to/from a flow cuvette 108. The continuous circulation of the fluorphage 12 is represented in the Figure by the arrow shown as "dye flow". An excitation beam 4 of the appropriate wavelength is then directed towards the fluorophage 12 within the cuvette 108. The excitation beam 4 exits the cuvette 108 towards an aluminium confocal mirror (f=150mm) 112 and then to a flat mirror 110. The beam 4 then passes through a phototransistor 114 and ultimately to a detector 116.

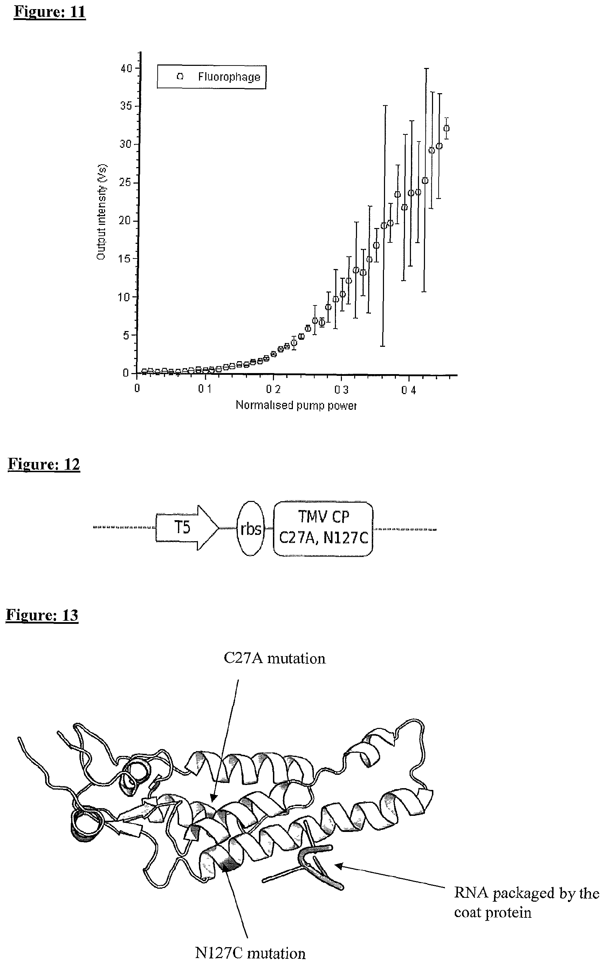

Threshold behaviour was observed, which is a characteristic property of a laser, as shown in Figure 11. Hence, the inventors have demonstrated that the fluorophage is clearly capable of lasing.

### Example 7 - Engineering an improved biological substrate for lasing

The fluorophage concept was demonstrated using M13 because it is a readily available, well-understood biological substrate, and it clearly showed that the M13 could act as a good biological substrate for producing a light-emitting (i.e. lasing) composition. However, the inventors then set out to show that other biological substrates could also be used. Using principles taken from synthetic biology, multiple biological components can be augmented to fabricate an improved biological substrate.

Dyes or light-emitting molecules can be attached to specific sites on the coat protein of a plant virus called Tobacco Mosaic Virus (TMV) such that there is no contact quenching between neighbouring dyes. This has inspired the design and fabrication of a recombinant Tobacco Mosaic Virus-like particle (rTMV) that can be expressed in *E*. *coli.*

Two genetic constructs were designed and constructed using synthetic genes and standard molecular cloning techniques. The first construct (shown in Figure 12) is a codon-optimised TMV coat protein gene with mutations at C27A and N127C with an IPTG inducable T5 promoter. The mutations introduce a unique attachment site for thiol-reactive molecular probes, including dyes and other light-emitting molecules. The crystal structure of the TMV coat protein is shown in Figure 13, and shows that the attachment sites are introduced near to the RNA binding site. For the coat protein to self-assemble into rods of controllable size, a packaging RNA transcript is required.

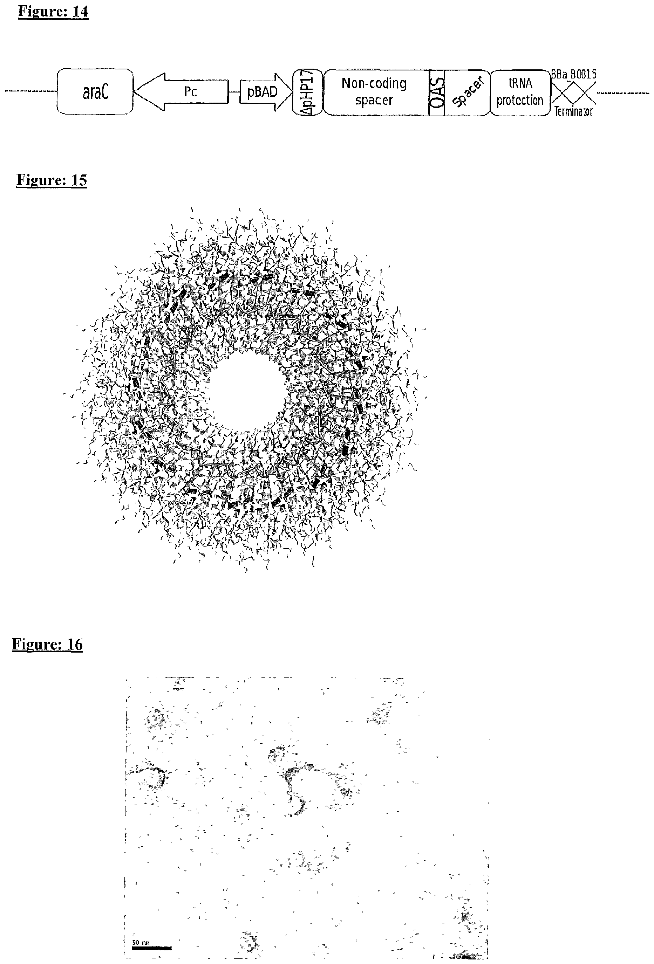

The second construct shown in Figure 14 is a gene containing no ribosome binding sites (rbs) with the TMV coat protein specific origin of assembly sequence flanked by 5' and 3' end protection sequences under the control of an L-arabinose induceable pBAD promoter. A 5' hairpin loop - pHP17 with the rbs removed - was incorporated for 5' end protection because it has been demonstrated to improve the half-life of RNA transcripts to nearly 20 min. The 3' end tRNA-like structure from the native TMV genome was included in this synthetic construct to offer 3' end protection. A strong terminator from the Register of Standard Biological Parts, BBa_B0015, ensures homogeneity in the length of RNA transcripts. Restriction sites are available for the cloning of non-coding spacer DNA which would increase the length of the rTMV. However, no spacer DNA was included in the example provided here.

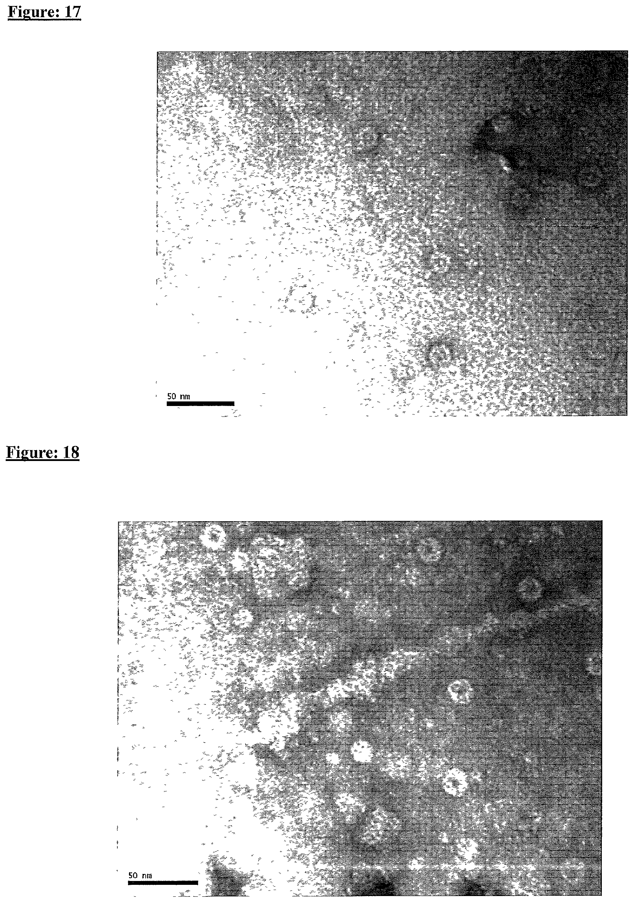

When co-expressed, the RNA transcript and the coat protein form rTMV. The crystal structure of the native virus (shown in Figure 15) shows that the attachment sites are on the underside of the protein after assembly. As such, dyes attached at these sites cannot interact with dyes attached in the layer above them because the protein sterically hinders this interaction. The helix between the proline at position 20 and the alanine at position 30 can also be seen to sterically hinder potential interactions between dyes attached to neighbouring coat proteins. Both constructs were transformed into *E. coli* Top 10 cells using standard techniques and single colonies were isolated. An overnight culture of these cells in Terrific Broth was grown overnight at 37 °C in a shaking incubator and was added to 50 ml expression medium in a baffled flask to a final concentration of 1%. The expression medium contained 36 g/L yeast extract, 18 g/L peptone, 2ml/L glycerol, 100 mM TRIS buffer pH7, 25 µg/ml ampicillin and 10 µg/ml kanamycin. After a three hour pre-induction phase at 37 °C in a shaking incubator at 250 rpm, L-arabinose and IPTG were added to final concentrations of 0.01% and 1 mM respectively. After six hours, cells were harvested and lysed overnight at 4 °C in a lysozyme, DNase I lysis solution. After centrifugation for 30 min at 10,400 x g (Eppendorf 5810 R) to remove the insoluble fraction of the lysate, the supernatant was subjected to a 4 g / 10 ml CsCl density gradient in an ultracentrifuge (Beckman Coulter Optima L-100 XP, Type 70.1 Ti rotor) at 35,000 rpm. A clear band was formed between the anticipated locations of the RNA and protein fractions, which was extracted. The density of this band was consistent with a RNA-protein complex, such as rTMV. After desalting in a PD-10 column (GE Healthcare), samples were inspected by transmission electron microscopy. rTMV rods can be readily identified by their characteristic 'o' shape (see Figures 16-18), confirming the successful fabrication of rTMV.

### Example 8 - rTMV fluorophage lasers

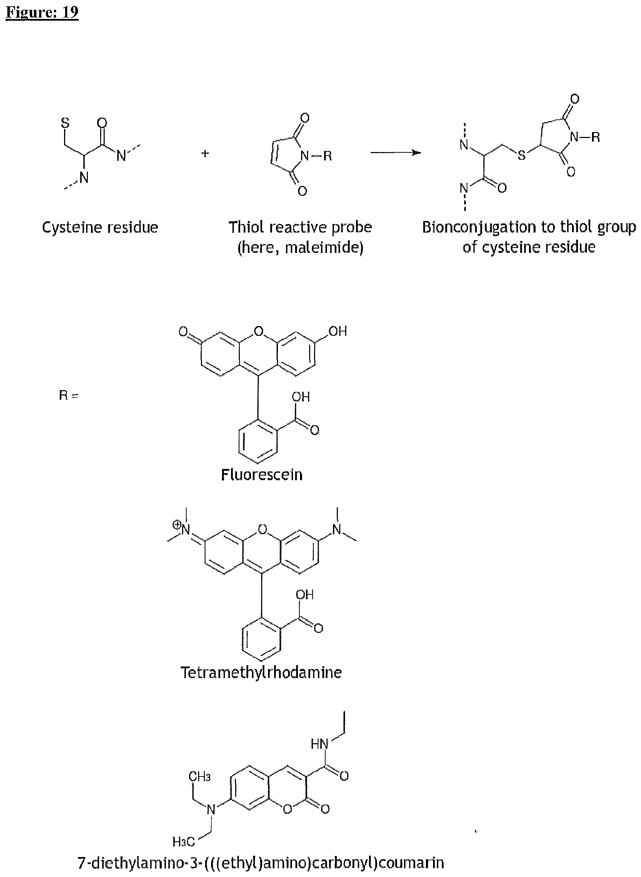

The inventors then used the following steps for the production of an rTMV fluorophage laser. Light emitting molecules, including dyes, having a thiol reactive moiety were attached to the cysteine residue. The reaction schemes are outlined in Figure 19. The Figure shows the reaaction between a cysteine residue on the rTMV and a thiol group on the probe (e.g. maleimide) in a bioconjugation reaction. Figure 19 shows three alternative thiol containing light-emitters, including fluorescein, tetramethylrhodamine and 7-diethylamino-3-(((ethyl)amino)carbonyl)coumarin. After purification in a size exclusion column or in a density gradient, the chemically modified rTMV was then tested for its lasing characteristics in the same way M13-fluorophage was tested in Example 6.

## Claims

1. A lasing composition comprising a proteinaceous biological substrate, wherein the biological substrate is chemically modified at specific attachment sites with light-emitting labels due to the presence of covalent bonds between the light-emitting labels and functional groups of amino acids present in the biological substrate, wherein the light-emitting labels comprise a fluorophore, the average molecular diameter of the light-emitting labels is between 0.5 nm and 2 nm, and the average distance between adjacent light-emitting labels is between 1 nm and 15 nm.

2. A lasing composition according to claim 1, wherein the distance between adjacent labels is such that they are unable to chemically react with each other, but can allow dipole-dipole interactions to occur between adjacent labels, and/or wherein the labels are attached to specific, spaced-apart attachment sites, which are disposed along the structure of the biological substrate, preferably wherein the attachment sites are amino acids, or a side chain thereof, and are regularly spaced apart along the substrate, and wherein the distance between adjacent potential attachment sites is substantially the same along the substrate and/or the positions of the potential attachment sites create a repeating pattern along the substrate, and wherein the attachment sites are not randomly arranged along the substrate.

3. A composition according to any preceding claim, wherein the average distance between adjacent light-emitting labels is between mm and 10nm, between mm and 5nm, or between mm and 3nm, and/or wherein the average distance between adjacent light-emitting labels is between 2nm and 5nm, or between 2nm and 3nm, and/or wherein the light-emitting labels are capable of absorbing between 220 nm and 1000 µm, or between 240 nm and 900 nm, or between 900 nm and 3000 nm, or between 460nm and 500nm, or between 3-8 µm (mid-infrared), or between 8-15 µm (long-infrared), or between 15-1000 µm (far-infrared).

4. A composition according to any preceding claim, wherein the light-emitting labels are members of the xanthene family of dyes, or other light-emitting molecules, such as GFP or quantum dots, and/or wherein the light-emitting labels comprise Rhodamine or a derivative thereof, and/or wherein the light-emitting labels comprise fluorescein or a derivative thereof, and/or wherein the lasing composition comprises one type or species of light-emitting label, and/or wherein more than one type or species of light-emitting labels are scaffolded to the substrate, and/or wherein the biological substrate comprises amino acid residues to which the light-emitting labels are attached, and/or wherein the biological substrate comprises a wild-type or mutant biological substrate, including a bacteriophage, actin fiber, biomimetic compound, or other proteinaceous substrate, and/or wherein the biological substrate comprises M13 filamentous bacteriophage (M13).

5. Use of a lasing composition as defined in any one of claims 1 to 4, as a laser dye.

6. A laser dye comprising the composition according to any one of claims 1-4.

7. Use of a lasing composition as defined in any one of claims 1 to 4, as a saturable absorber.

8. A laser comprising a lasing composition as defined in any one of claims 1 to 4.

9. A laser according to claim 8, wherein the laser is selected from the group of lasers consisting of: a plasmonic laser; a single-particle plasmonic laser; a dye laser; a solid state laser; an electrically pumped solid state laser; a liquid dye laser; a tunable laser; a pulse laser; and an ultrashort pulse laser, and/or wherein the laser is a dye laser.

10. In vitro use of a lasing composition as defined in any one of claims 1 to 4, as a biosensor, or in biological imaging applications.

11. A liquid crystal structure or film comprising a lasing composition as defined in any one of claims 1 to 4.

12. Use of the liquid crystal structure or film according to claim 11, as a display.

13. A display comprising a lasing composition as defined in any one of claims 1 to 4.

14. A display according to claim 13, wherein the display is for a computation, control or communications device.

15. An optical computer comprising a lasing composition as defined in any one of claims 1 to 4.

## Patentansprüche

1. Lasemde Zusammensetzung, umfassend ein proteinhaltiges biologisches Substrat, wobei das biologische Substrat an spezifischen Anlagerungsstellen mit lichtemittierenden Markierungen chemisch modifiziert ist, aufgrund der Anwesenheit von kovalenten Bindungen zwischen den lichtemittierenden Markierungen und funktionellen Gruppen von Aminosäuren in dem biologischen Substrat, wobei die lichtemittierenden Markierungen einen Fluorophor umfassen, der durchschnittliche molekulare Durchmesser der lichtemittierenden Markierungen zwischen 0,5 nm und 2 nm ist und der durchschnittliche Abstand zwischen benachbarten lichtemittierenden Markierungen zwischen 1 nm und 15 nm ist.

2. Lasemde Zusammensetzung nach Anspruch 1, wobei der Abstand zwischen benachbarten Markierungen derart ist, dass sie nicht in der Lage sind, chemisch miteinander zu reagieren, aber ermöglichen können, dass Dipol-Dipol-Wechselwirkungen zwischen benachbarten Markierungen auftreten können, und/oder wobei die Markierungen an spezifischen, beabstandeten Anlagerungsstellen angelagert sind, die entlang der Struktur des biologischen Substrats angeordnet sind, vorzugsweise wobei die Anlagerungsstellen Aminosäuren oder eine Seitenkette davon sind, und regelmäßig entlang des Substrats beabstandet sind, und wobei der Abstand zwischen benachbarten potenziellen Anlagerungsstellen im Wesentlichen entlang des Substrats gleich ist und/oder die Positionen der potenziellen Anlagerungsstellen ein sich wiederholendes Muster entlang des Substrats erzeugen, und wobei die Anlagerungsstellen nicht zufällig entlang des Substrats angeordnet sind.

3. Zusammensetzung nach einem vorherigen Anspruch, wobei der durchschnittliche Abstand zwischen benachbarten lichtemittierenden Markierungen zwischen 1 nm und 10 nm, zwischen 1 nm und 5 nm oder zwischen 1 nm und 3 nm ist, und/oder wobei der durchschnittliche Abstand zwischen benachbarten lichtemittierenden Markierungen zwischen 2 nm und 5 nm oder zwischen 2 nm und 3 nm ist, und/oder wobei die lichtemittierenden Markierungen in der Lage sind, zwischen 220 nm und 1000 µm oder zwischen 240 nm und 900 nm oder zwischen 900 nm und 3000 nm oder zwischen 460 nm und 500 nm oder zwischen 3-8 µm (Mittelinfrarot) oder zwischen 8-15 µm (Langinfrarot) oder zwischen 15-1000 µm (Ferninfrarot) zu absorbieren.

4. Zusammensetzung nach einem der vorherigen Ansprüche, wobei die lichtemittierenden Markierungen Mitglieder der Xanthen-Familie von Farbstoffen oder anderen lichtemittierenden Molekülen, wie z. B. GFP oder Quantenpunkten, sind, und/oder wobei die lichtemittierenden Markierungen Rhodamin oder ein Derivat davon umfassen, und/oder wobei die lichtemittierenden Markierungen Fluorescein oder ein Derivat davon umfassen, und/oder wobei die lasernde Zusammensetzung eine Art oder Spezies von lichtemittierender Markierung umfasst, und/oder wobei mehr als eine Art oder Spezies von lichtemittierenden Markierungen auf dem Substrat eingerüstet sind, und/oder wobei das biologische Substrat Aminosäurereste umfasst, an denen die lichtemittierenden Markierungen angelagert sind, und/oder wobei das biologische Substrat ein biologisches Wildtyp- oder Mutant-Substrat umfasst, einschließlich eines Bakteriophagen, einer Aktinfaser, einer biomimetischen Verbindung oder eines anderen proteinhaltigen Substrats, und/oder wobei das biologische Substrat den filamentösen Bakteriophagen M13 (M13) umfasst.

5. Verwendung einer lasernden Zusammensetzung nach einem der Ansprüche 1 bis 4 als ein Laserfarbstoff.

6. Laserfarbstoff, umfassend die Zusammensetzung nach einem der Ansprüche 1-4.

7. Verwendung einer lasernden Zusammensetzung nach einem der Ansprüche 1 bis 4 als sättigbarer Absorber.

8. Laser, umfassend eine lasernde Zusammensetzung nach einem der Ansprüche 1 bis 4.

9. Laser nach Anspruch 8, wobei der Laser ausgewählt ist aus der Laser-Gruppe bestehend aus: einem plasmonischen Laser; einem plasmonischen Einzelpartikellaser; einem Farbstofflaser; einem Festkörperlaser; einem elektrisch gepumpten Festkörperlaser; einem Flüssigfarbstofflaser; einem abstimmbaren Laser; einem Impulslaser; und einem Ultrakurzimpulslaser, und/oder wobei der Laser ein Farbstofflaser ist.

10. In-vitro-Verwendung einer lasernden Zusammensetzung nach einem der Ansprüche 1 bis 4 als Biosensor oder in biologischen Bildgebungsanwendungen.

11. Flüssigkristallstruktur oder Flüssigkristallfilm, umfassend eine lasernde Zusammensetzung nach einem der Ansprüche 1 bis 4.

12. Verwendung der Flüssigkristallstruktur oder des Flüssigkristallfilms nach Anspruch 11 als eine Anzeige.

13. Anzeige, umfassend eine lasernde Zusammensetzung nach einem der Ansprüche 1 bis 4.

14. Anzeige nach Anspruch 13, wobei die Anzeige für eine Rechen-, Steuer- oder Kommunikationsvorrichtung ist.

15. Optischer Computer, umfassend eine lasernde Zusammensetzung nach einem der Ansprüche 1 bis 4.

## Revendications

1. Composition laser comprenant un substrat biologique protéique, dans laquelle le substrat biologique est chimiquement modifié au niveau de sites d'attachement spécifiques avec des marqueurs électroluminescents du fait de la présence de liaisons covalentes entres les marqueurs électroluminescents et des groupes fonctionnels d'acides aminés présents dans le substrat biologique, dans laquelle les marqueurs électroluminescents comprennent un fluorophore, le diamètre moléculaire moyen des marqueurs électroluminescents est entre 0,5 nm et 2 nm, et la distance moyenne entre des marqueurs électroluminescents adjacents est entre 1 nm et 15 nm.

2. Composition laser selon la revendication 1, dans laquelle la distance entre des marqueurs adjacents est telle qu'ils sont incapables de réagir chimiquement les uns avec les autres, mais peuvent permettre la production d'interactions dipôle-dipôle entre des marqueurs adjacents, et/ou dans laquelle les marqueurs sont attachés à des sites d'attachement éloignés spécifiques, qui sont disposés le long de la structure du substrat biologique, de préférence dans laquelle les sites d'attachement sont des acides aminés, ou une chaîne latérale de ceux-ci, et sont espacés régulièrement le long du substrat, et dans laquelle la distance entre des sites d'attachement potentiels adjacents est sensiblement la même le long du substrat et/ou les positions des sites d'attachement potentiels créent un motif répétitif le long du substrat, et dans laquelle les sites d'attachement ne sont pas disposés de manière aléatoire le long du substrat.

3. Composition selon l'une quelconque des revendications précédentes, dans laquelle la distance moyenne entre des marqueurs électroluminescents adjacents est entre 1 nm et 10 nm, entre 1 nm et 5 nm, ou entre 1 nm et 3 nm, et/ou dans laquelle la distance moyenne entre des marqueurs électroluminescents adjacents est entre 2 nm et 5 nm, ou entre 2 nm et 3 nm, et/ou dans laquelle les marqueurs électroluminescents sont en mesure d'absorber entre 220 nm et 1000 µm, ou entre 240 nm et 900 nm, ou entre 900 nm et 3000 nm, ou entre 460 nm et 500 nm, ou entre 3-8 µm (infrarouge moyen), ou entre 8-15 µm (infrarouge long), ou entre 15-1000 µm (infrarouge lointain).

4. Composition selon l'une quelconque des revendications précédentes, dans laquelle les marqueurs électroluminescents sont membres de la famille xanthène de colorants, ou d'autres molécules électroluminescentes, telles que des GFP ou boîtes quantiques, et/ou dans laquelle les marqueurs électroluminescents comprennent de la Rhodamine ou un dérivé de celle-ci, et/ou dans laquelle les marqueurs électroluminescents comprennent une fluorescéine ou un dérivé de celle-ci, et/ou dans laquelle la composition laser comprend un type ou une espèce de marqueur électroluminescent, et/ou dans laquelle plus d'un type ou d'une espèce de marqueurs électroluminescents sont étayés au substrat, et/ou dans laquelle le substrat biologique comprend des résidus d'acides aminés auxquels les marqueurs électroluminescents sont attachés, et/ou dans laquelle le substrat biologique comprend un substrat biologique mutant ou de type sauvage, incluant un bactériophage, une fibre actine, un composé biomimétique, ou un autre substrat protéique, et/ou dans laquelle le substrat biologique comprend un bactériophage filamenteux M13 (M13).

5. Application d'une composition laser telle que définie dans l'une quelconque des revendications 1 à 4, en tant que laser à colorant.

6. Laser à colorant comprenant la composition selon l'une quelconque des revendications 1 à 4.

7. Application d'une composition laser telle que définie dans l'une quelconque des revendications 1 à 4, en tant qu'absorbeur saturable.

8. Laser comprenant une composition laser selon l'une quelconque des revendications 1 à 4.

9. Laser selon la revendication 8, dans lequel le laser est sélectionné parmi le groupe de lasers constitué de : un laser plasmonique ; un laser plasmonique mono-particule ; un laser à colorant ; un laser à état solide ; un laser à état solide à pompage électrique ; un laser à colorant liquide ; un laser accordable ; un laser à impulsion ; et un laser à impulsion ultracourte, et/ou dans lequel le laser est un laser à colorant.

10. Application in-vitro d'une composition laser telle que définie dans l'une quelconque des revendications 1 à 4, en tant que biocapteur, ou dans des applications d'imagerie biologique.

11. Structure ou film à cristaux liquides comprenant une composition laser selon l'une quelconque des revendications 1 à 4.

12. Application d'une structure ou d'un film à cristaux liquides selon la revendication 11, en tant qu'afficheur.

13. Afficheur comprenant une composition laser selon l'une quelconque des revendications 1 à 4.

14. Afficheur selon la revendication 13, dans lequel l'afficheur est destiné à un dispositif de calcul, commande ou communication.

15. Ordinateur optique comprenant une composition laser selon l'une quelconque des revendications 1 à 4.
